# EUROPEAN PATENT SPECIFICATION

(11) **EP 3 939 547 B1**
(45) Date of publication and mention of the grant of the patent: **08.11.2023**
(21) Application number: 20857064.8
(22) Date of filing: 19.05.2020
(51) Int. Cl.: A61F 2/44, A61F 2/46, A61F 2/30

(54) **SYSTEM FOR GUIDING INTERBODY SPACER BETWEEN VERTEBRAL BODIES**
SYSTEM ZUR FÜHRUNG DES ZWISCHENKÖRPERDISTANZSTÜCKS ZWISCHEN WIRBELKÖRPERN
SYSTÈME POUR GUIDER UN ESPACEUR INTERVERTÉBRAL ENTRE DES CORPS VERTÉBRAUX

(30) Priority: 30.08.2019 JP 2019157595
(43) Date of publication of application: 19.01.2022
(73) Proprietor: Liga Ace Corporation, Shizuoka City, Shizuoka 420-0886 (JP)
(72) Inventor: SHINOZAKI Yoshio, Shizuoka City, Shizuoka 420-0886 (JP)
(74) Representative: VKK Patentanwälte PartG mbB
(86) International application number: PCT/JP2020/019786
(87) International publication number: WO 2021/038982

(56) References cited:
- JP-A- 2004 209 249
- JP-A- 2007 306 980
- JP-A- 2012 523 928
- JP-A- 2017 000 753
- US-A1- 2002 045 904

## Description

### Technical Field

The present invention pertains to a system for guiding an interbody spacer between vertebral bodies.

### Background Art

For example, it is essential to use an interbody spacer for obtaining bony fusion by means of PLIF (Posterior Lumbar Interbody Fusion), and it is possible to further stabilize the intervertebral space and achieve firmer fusion by inserting a spacer which is as large as possible and conforms to the shape of an intervertebral space. In addition, a larger spacer is expected to be capable of being filled with a greater quantity of graft bone, etc., and is considered to contribute to future intervertebral bony fusion.

There is concern that the insertion of an interbody spacer only from a unilateral side by using a conventional box-type interbody spacer by means of a unilateral approach and unilateral facetectomy may cause contralateral intervertebral instability to remain, which may be detrimental to bony fusion. There is also concern that the insertion of a box-type interbody spacer from bilateral sides by means of a bilateral approach and bilateral facetectomy may further induce intervertebral instability. In contrast, the insertion of an interbody spacer or the insertion of a conventional boomerang-shaped interbody spacer to the contralateral side by means of a unilateral approach and unilateral facetectomy is a reasonable method as it is possible to provide support as far as the contralateral side while preserving contralateral intervertebral joints.

### Prior Art Documents

### Patent Documents

Patent document US2002/045904,
Patent Document 1: JP 2012-532693A
Patent Document 2: JP 2016-512107A
Patent Document 3: JP 2019-517891A

### Summary of the Invention

### Problem to Be Solved by the Invention

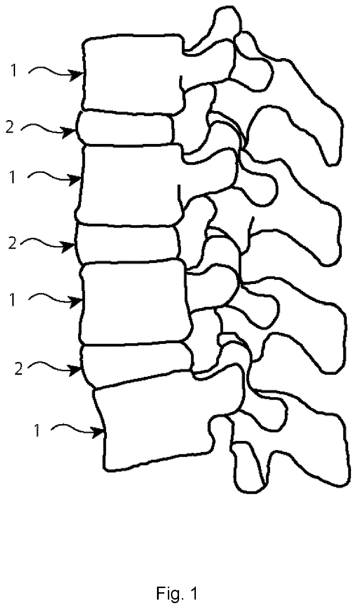

When inserting and placing an interbody spacer between vertebral bodies, fenestration is performed in the annulus fibrosus of an intervertebral disc 2 between vertebral bodies 1 shown in Fig. 1, and the nucleus pulposus therein is removed, and then the interbody spacer is inserted and placed in the intervertebral space after the removal. As the spinal nervous system and blood vessels run near the intervertebral disc 2, extreme care is required when carrying out the surgical operation.

As the spinal column performs the function of supporting body weight, an interbody spacer is required to have high rigidity and to cover a large area between vertebral bodies, and furthermore, as the surgical field is narrow and there are the nervous system and blood vessels running nearby, skills and surgical systems are required for placing the interbody spacer in an appropriate position while avoiding the nervous system and blood vessels.

As mentioned above, the insertion of a conventional boomerang-shaped interbody spacer is a reasonable method because it is possible to provide support as far as the contralateral side while preserving contralateral intervertebral joints. However, use of an intraoperative fluoroscopic device and highly advanced techniques are required when sending the interbody spacer to the contralateral side.

In addition, regarding a conventional interbody spacer, the size of the interbody spacer that can be inserted through a narrow insertion opening is limited. For this reason, interbody spacers having flexibility and insertion tools and the like that can be oriented within the intervertebral space have been developed to date, but many of those systems are structurally complicated and thereby have vulnerability, and thus few systems have been put into a practical use.

In the system described in Patent Document 1, interbody spacers are connected to each other by a flexible bridge. The mechanism is configured such that the interbody spacers are inserted into an inserter tube, allowed to pass through the inserter tube to be inserted and placed between the vertebral bodies. The structures of the interbody spacers are complicated, which reduce the rigidity of the interbody spacers. Further, in the disclosed embodiments, the insertion tool includes a guide rail for guiding the bottom surface of each interbody spacer and a guide rail for guiding the bottom surface, superior surface, and side surface of each interbody spacer, and thus there remains a problem to be solved in guiding the interbody spacer to a narrow surgical field.

In the system described in Patent Document 2, an interbody spacer has an expandable function. The system is configured such that after placing the interbody spacer between vertebral bodies in a non-expanded state by using a tool, the interbody spacer is expanded within the intervertebral space. Numerous parts are connected by pins, etc. in the interbody spacer, so that the structure is complicated, and the rigidity is reduced. In addition, it is required that the interbody spacer be placed in a non-expanded state between the vertebral bodies and then expanded, and thus the system is complicated and a highly advanced skill of a surgeon is required in surgical operation.

In the system described in Patent Document 3, an interbody spacer is one integrated piece and has rigidity. A tool for placing the interbody spacer includes a gripping portion for gripping a pin that is provided in the interbody spacer, and a surgeon is required to place the interbody spacer in a narrow surgical field while adjusting the orientation thereof, and accordingly there is a problem that the surgical operation is difficult.

It is an object of the present invention, as claimed in claim 1, to provide a simple system capable of easily placing an interbody spacer accurately in a predetermined position between vertebral bodies even in a narrow surgical field.

### Means for Solving the Problem

The present invention (1) is a system for treating a spinal disease: an interbody spacer which is curved in a horizontal direction and used by being inserted between vertebral bodies, the interbody spacer including a pair of contact surfaces to be in contact with each of the vertebral bodies, and including a ventral surface which is a side surface on a ventral side having a convex shape in plan view and which connects the pair of contact surfaces on the ventral side, and a dorsal surface which is a side surface on a dorsal side having a concave shape in plan view and which connects the pair of contact surfaces on the dorsal side, the interbody spacer including an engagement portion, which is located along the direction in which the interbody spacer is curved, in the ventral surface and/or the dorsal surface; and a guiding tool for guiding the interbody spacer to a predetermined position between the vertebral bodies, the guiding tool including, on the distal end side, a guide rail portion to be fitted into either one of the engagement portion in the ventral surface and the engagement portion in the dorsal surface, the guide rail portion having a radius of curvature in plan view which is substantially the same as the radius of curvature of the ventral surface or the dorsal surface in plan view; wherein either one of the transverse cross-section of the engagement portion and the transverse cross-section of the guide rail portion has a concave shape, while the other has a convex shape that corresponds to the concave shape, and the concave shape includes a portion having a maximum width that is greater than the opening width of the concave shape that is opposed to the convex shape.

In the system of the present invention (1), the engagement portion is provided along the direction in which the interbody spacer is curved in the ventral surface and/or the dorsal surface of the interbody spacer. The guiding tool includes, on the distal end side, the guide rail portion to be fitted into either one of the engagement portion in the ventral surface and the engagement portion in the dorsal surface. The radius of curvature of the guide rail portion in plan view is substantially the same as the radius of curvature of the ventral surface or the dorsal surface in plan view, so that the interbody spacer can be moved along the guide rail portion. Once the guiding tool is placed in a predetermined position, it is possible to guide the interbody spacer accurately and easily to a predetermined position between vertebral bodies.

By using the present system, for example, when the insertion is performed by a TLIF (Transforaminal Lumbar Interbody Fusion) approach, rotation is made approximately around the dura mater, and insertion is made from the lateral edge of the intervertebral space to be in contact with the anterior edge and the lateral edge on the contralateral side. By inserting the interbody spacer along the guide rail portion of the guiding tool that is placed in advance between the vertebral bodies, it is possible to insert the interbody spacer safely to the optimal position on the contralateral side more accurately and easily.

### Effect of the Invention

According to the system of the present invention, the system has a simple configuration, and thus it is possible to easily place the interbody spacer accurately in a predetermined position between vertebral bodies even in a narrow surgical field.

### Brief Description of the Drawings

[Fig. 1] shows a side view of the spine (the lumbar spine).
[Fig. 2] shows a perspective view in which a guiding tool is placed between vertebral bodies.
[Fig. 3] shows a perspective view of an interbody spacer of Example 1 according to the present invention.
[Fig. 4] shows a plan view, a medial side view, a lateral side view, and a ventral side view of the interbody spacer of Example 1 according to the present invention.
[Fig. 5] shows a perspective view of the guiding tool of Example 2 according to the present invention.
[Fig. 6] shows a plan view of the guiding tool of Example 2 according to the present invention.
[Fig. 7] shows a perspective view of the guiding tool of Example 3 according to the present invention.
[Fig. 8] shows a plan view of the guiding tool of Example 3 according to the present invention.
[Fig. 9] schematically shows the manner in which two interbody spacers according to the present invention are symmetrically placed between vertebral bodies, by engaging the inner periphery of the guide rail portion of the guiding tool of Example 2 with the ventral surface of each of the interbody spacers.
[Fig. 10] schematically shows the manner in which two interbody spacers according to the present invention are symmetrically placed between vertebral bodies, by engaging the outer periphery of the guide rail portion of the guiding tool of Example 3 with the dorsal surface of each of the interbody spacers.
[Fig. 11] schematically shows specific examples of the engagement state between the interbody spacer and the guide rail portion of the guiding tool of the system according to the present invention.

### Embodiment for Carrying Out the Invention

The embodiment for carrying out the present invention will now be explained with reference to the drawings. In the following explanations, the superior and inferior denote an upper side and a lower side in the drawings, respectively. These "superior and inferior" are used for the sake of convenience, and upon placement, the superior and inferior may be reversed or the positioning may be horizontal.

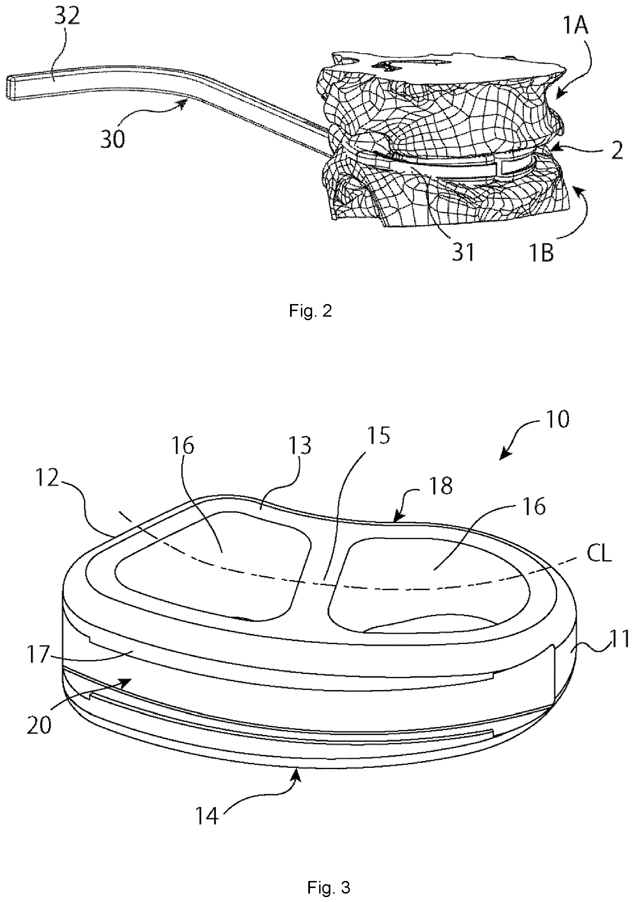

Fig. 2 shows a schematic view of the state in which a guide rail portion 31 of a guiding tool 30 is inserted and placed in the intervertebral space between a cranial vertebral body 1A and a caudal vertebral body 1B, which is viewed from the ventral side. The operator grips the gripping portion 32 and performs insertion. Actually, the intervertebral space between the cranial vertebral body 1A and the caudal vertebral body 1B is surrounded by tissue called an annulus fibrosus and thus cannot be seen from the ventral side, but the annulus fibrosus is omitted so that the state of placement can be recognized.

Fig. 3 shows a perspective view of Example 1 of the interbody spacer according to the present invention, which is viewed from the ventral side. A superior surface 13 is in contact with the caudal surface of the cranial vertebral body 1A, while an inferior surface 14 is in contact with the cranial surface of the caudal vertebral body 1B. A lateral surface 11 and a medial surface 12 are located at both end portions along the central axis CL. The overall shape is such that the ventral side is convexly curved. A side surface on the ventral side is a ventral surface 17, and a concaved side surface is a dorsal surface 18. The superior surface 13 and the inferior surface 14 are carved with grooves or the like for having the superior surface 13 and the inferior surface 14 firmly joined to the cranial vertebral body 1A and caudal vertebral body 1B, respectively, but these grooves or the like are omitted in Fig. 3.

The interbody spacer 10 of Example 1 has two holes penetrating the superior surface 13 and the inferior surface 14, and these two holes are partitioned by a middle section 15. These holes are called bone-graft sites 16 and are used to be filled with a highly osteophilic material such as patient's autogenous bone or artificial bone. An engagement portion 20 to be engaged with a guiding tool described later is formed in the ventral surface 17 in Fig. 3. The number of these holes is not limited to two, and these holes are not essential in the present invention, and the interbody spacer may be one without any hole.

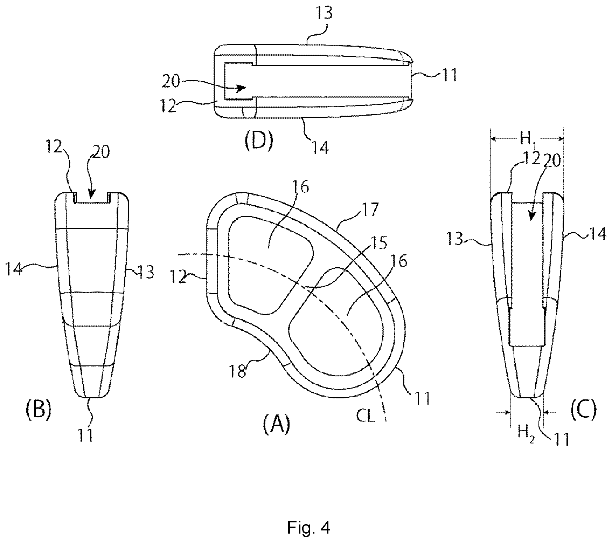

Fig. 4 shows a plan view, a medial side view, a lateral side view, and a ventral side view of the interbody spacer 10 of Example 1. Fig. 4(A) is the plan view, Fig. 4(B) is the medial side view, Fig. 4(C) is the lateral side view, and Fig. 4(D) is the ventral side view. H₁ in the drawing is the height of the ventral surface 17, and H₂ is the height of the dorsal surface 18.

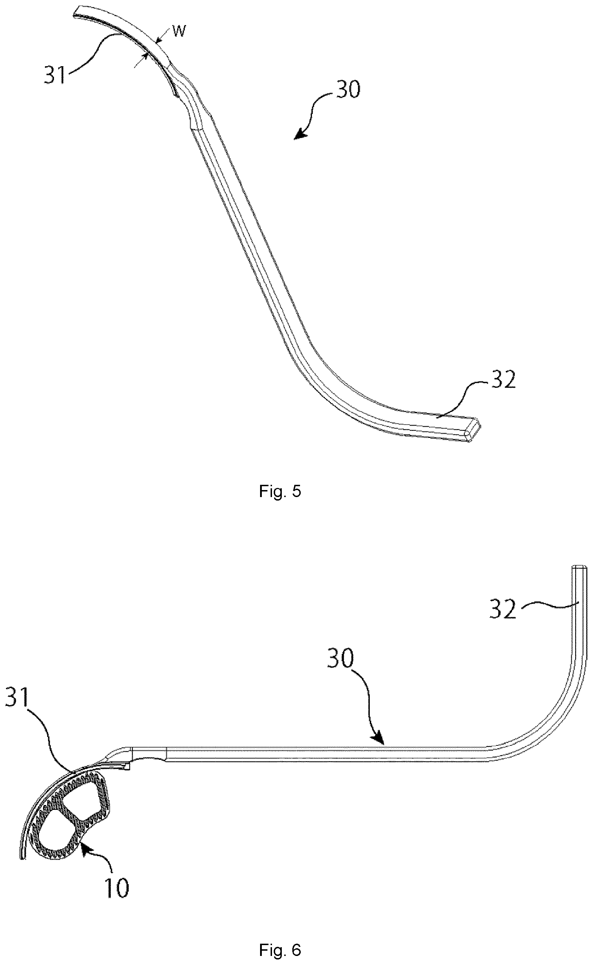

Fig. 5 shows a perspective view of the guiding tool 30 of Example 2, and Fig. 6 shows a plan view of the guiding tool 30 of Example 2. A guide rail portion 31 to be engaged with the engagement portion 20 of the interbody spacer 10, and a gripping portion 32 to be gripped by the operator are formed in the guiding tool 30. Fig. 6 shows a state in which the engagement portion 20 formed in the ventral surface 17 of the interbody spacer 10 is engaged with the guide rail portion 31. W in Fig. 5 denotes the transverse width of the guide rail portion 31.

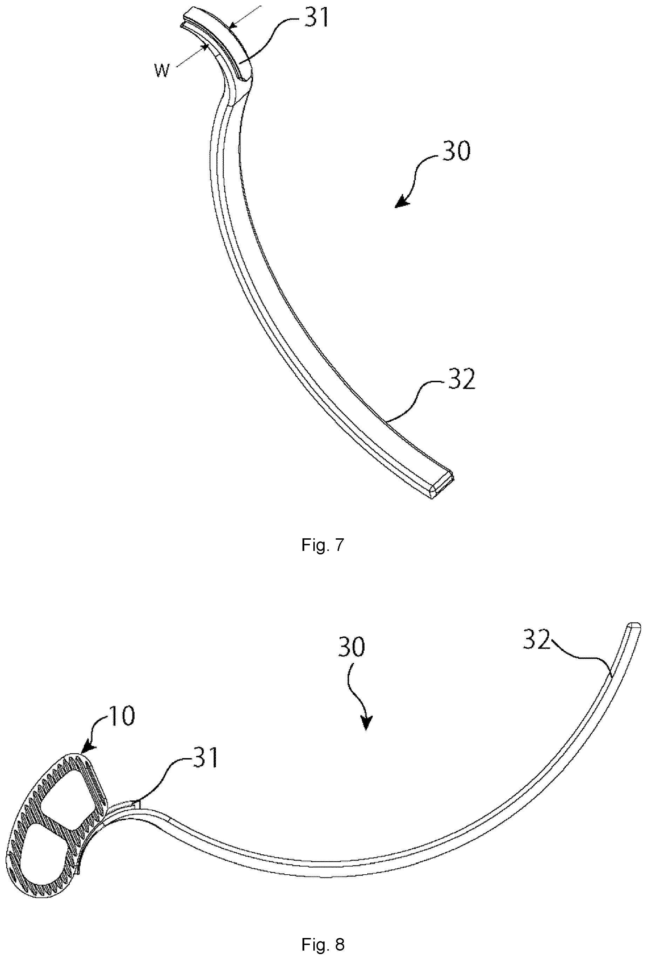

Fig. 7 shows a perspective view of the guiding tool 30 of Example 3, and Fig. 8 shows a plan view of the guiding tool 30 of Example 3. The guide rail portion 31 to be engaged with the engagement portion 20 of the interbody spacer 10, and the gripping portion 32 to be gripped by the operator are formed in the guiding tool 30. Fig. 8 shows a state in which the engagement portion 20 formed in the dorsal surface 18 of the interbody spacer 10 is engaged with the guide rail portion 31. W in Fig. 7 denotes the transverse width of the guide rail portion 31.

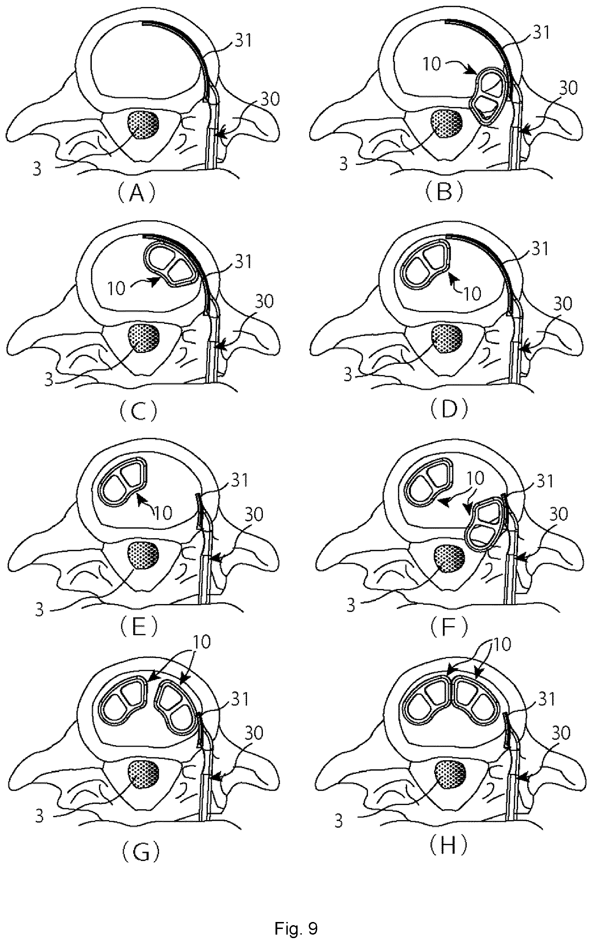

Fig. 9 schematically shows the steps of placing two interbody spacers 10 between vertebral bodies using the guiding tool 30 of Example 2. In Fig. 9(A), the guiding tool 30 is placed in a predetermined position while avoiding nerves 3. In Fig. 9(B), the engagement portion 20 of the ventral surface 17 on the lateral surface 11 side of the interbody spacer 10 is engaged with the guide rail portion 31. In Fig. 9(C), a pusher (not shown) is used to push the medial surface 12 side to insert the interbody spacer 10 along the guide rail portion 31, and in Fig. 9(D), the first one of the interbody spacers 10 is placed in a predetermined position.

Thereafter, in Fig. 9(E), the guiding tool 30 is pulled out and a new guiding tool 30 with a shorter guide rail portion 31 is set in a predetermined position between the vertebral bodies. In Fig. 9(F), the engagement portion 20 of the ventral surface 17 on the medial surface 12 side of the interbody spacer 10 is engaged with the guide rail portion 31. In Fig. 9(G), a pusher (not shown) is used to push the lateral surface 11 side to insert the interbody spacer 10 along the guide rail portion 31, and in Fig. 9(H), the second one of the interbody spacers 10 is placed in a predetermined position, and then the guiding tool 30 is pulled out.

The guide rail portion 31 of Example 2 is placed against the annulus fibrosus of the intervertebral disc on the ventral side of intervertebral space so as to serve as a wall on the ventral side, so that it is possible to prevent the interbody spacer from perforating the annulus fibrosus and being dislocated to the ventral side from the intervertebral space.

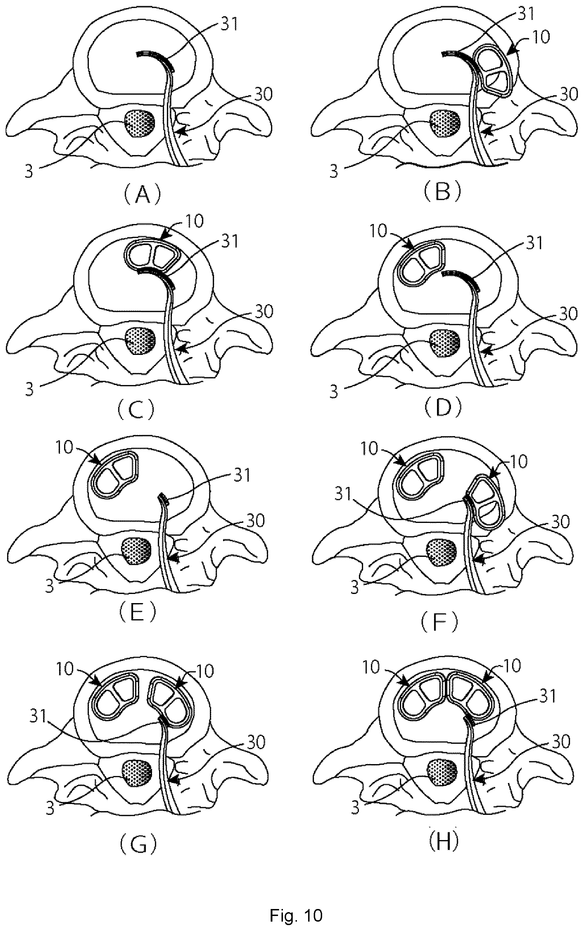

Fig. 10 schematically shows the steps of placing two interbody spacers 10 between vertebral bodies using the guiding tool 30 of Example 3. The guiding tool 30 of Example 3 is configured to engage with the engagement portion 20 formed in the dorsal surface 18 of the interbody spacer 10, which differs from the guiding tool 30 of Example 2. With this configuration, the nerves 3 are protected by the guiding tool 30.

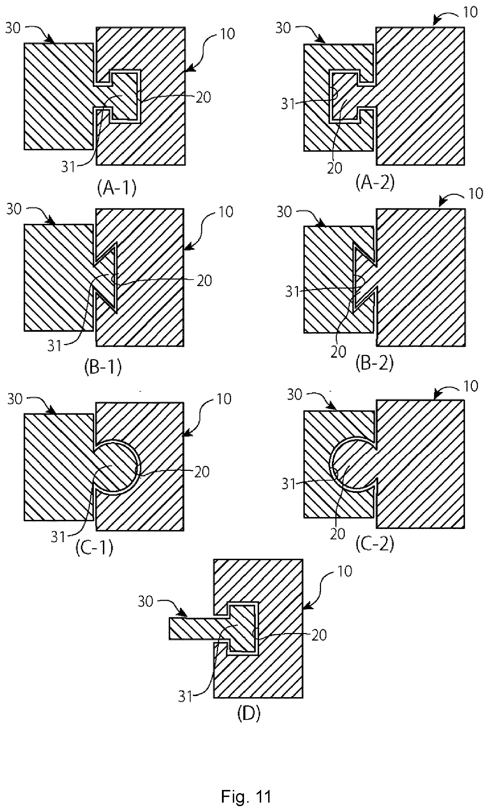

Fig. 11 schematically shows specific examples of the engagement state between the interbody spacer 10 and the guide rail portion 31 of the guiding tool 30. In Fig. 11(A-1), the cross-section of the guide rail portion 31 is a T-shaped convex, and the cross-section of the opposing engagement portion 20 of the interbody spacer 10 is a T-shaped concave. Fig. 11(A-2) shows the reverse of Fig. 11(A-1).

Fig. 11(B-1) and Fig. 11(B-2) show an engagement structure of a dovetail groove, and Fig. 11(C-1) and Fig. 11(C-2) show an engagement structure having a ball-and-socket relation. These engagements have their own characteristics, and the T-shape and the dovetail groove shape are characterized in having an advantage in the processing cost and capability of secure retention, while the ball-and-socket shape has a better sliding property.

Fig. 11(D) is a modification of Fig. 11(A-1), in which the transverse width of the guide rail portion 31 is extremely small compared to the height of the ventral surface and/or the dorsal surface of the interbody spacer, and the interbody spacer 10 can be more easily and accurately placed in a predetermined position even in a narrow area between vertebral bodies.

The present international application claims priority based on Japanese Patent Application No. 2019-157595 filed on August 30, 2019.

### Industrial Applicability

The system according to the present invention is a simple system, and using this system makes it possible to easily place an interbody spacer accurately in a predetermined position between vertebral bodies even in a narrow surgical field. Descriptions of Reference Numerals

1: vertebral body
1A: cranial vertebral body
1B: caudal vertebral body
2: intervertebral disc
3: nerves
10: interbody spacer
11: lateral surface
12: medial surface
13: superior surface
14: inferior surface
15: middle section
16: bone-graft site
17: ventral surface
18: dorsal surface
20: engagement portion
30: guiding tool
31: guide rail portion
32: gripping portion

## Claims

1. A system for treating a spinal disease, said system comprising:
an interbody spacer which is curved in a horizontal direction and used by being inserted between vertebral bodies,
the interbody spacer (10) including a pair of contact surfaces to be in contact with each of the vertebral bodies, and
including a ventral surface which is a side surface on a ventral side having a convex shape in plan view and which connects said pair of contact surfaces on the ventral side, and
a dorsal surface which is a side surface on a dorsal side having a concave shape in plan view and which connects said pair of contact surfaces on the dorsal side,
the interbody spacer including an engagement portion (20) which is located along the direction in which the interbody spacer is curved, in the ventral surface and/or the dorsal surface;
and a guiding tool (30) for guiding the interbody spacer to a predetermined position between the vertebral bodies, the guiding tool including, on a distal end side, a guide rail portion (31) said guide rail portion having a radius of curvature in plan view which is the same as a radius of curvature of the ventral surface or the dorsal surface in plan view; **characterized in that**
the guide rail portion is designed in such a way to be fitted into either one of the engagement portion in the ventral surface and the engagement portion in the dorsal surface,
wherein either one of the transverse cross-section of the engagement portion and the transverse cross-section of the guide rail portion has a concave shape, while the other has a convex shape that corresponds to the concave shape, and the concave shape includes a portion having a maximum width that is greater than an opening width of the concave shape that is opposed to the convex shape.

2. The system according to Claim 1, wherein the guide rail portion has a transverse width equal to or smaller than the height of the ventral surface and/or the dorsal surface of the interbody spacer.

3. The system according to Claim 1 or 2, **characterized in that** the transverse cross-section of the engagement portion is a T-shaped concave, and the transverse cross-section of the guide rail portion is s T-shaped convex.

4. The system according the Claim 1 or 2, **characterized in that** the transverse cross-section of the engagement portion is a T-shaped convex, and the transverse cross-section of the guide rail portion is a T-shaped concave.

5. The interbody spacer according to any one of Claims 1 to 4, the engagement portion having a radius of curvature in plan view which is the same as a radius of curvature of the ventral surface or the dorsal surface in plan view.

6. The guiding tool according to any one of Claims 1 to 4.

## Patentansprüche

1. System zur Behandlung einer spinalen Erkrankung, wobei das genannte System umfasst:
ein Zwischenkörperdistanzstück, das in einer horizontalen Richtung gekrümmt ist und verwendet wird, indem es zwischen Wirbelkörpern eingesetzt wird,
wobei das Zwischenkörperdistanzstück (10) ein Paar von Kontaktflächen aufweist, um mit jedem der Wirbelkörper in Kontakt zu stehen, und
eine ventrale Fläche aufweist, die eine Seitenfläche auf einer ventralen Seite mit einer konvexen Form in der Draufsicht ist und die das genannte Paar von Kontaktflächen auf der ventralen Seite verbindet, und
eine dorsale Fläche, die eine Seitenfläche auf einer dorsalen Seite mit einer konkaven Form in der Draufsicht ist und die das genannte Paar von Kontaktflächen auf der dorsalen Seite verbindet,
wobei Zwischenkörperdistanzstück einen Eingriffsabschnitt (20) aufweist, der lokalisiert ist entlang der Richtung, in der das Zwischenkörperdistanzstück gekrümmt ist, in der ventralen und/oder der dorsalen Fläche;
und ein Führungswerkzeug (30) zum Führen des Zwischenkörperdistanzstücks zu einer vorherbestimmten Position zwischen den Wirbelkörpern,
wobei das Führungswerkzeug, auf einer distalen Endseite, einen Führungsschienenabschnitt (31) aufweist,
wobei der genannte Führungsschienenabschnitt einen Krümmungsradius in der Draufsicht hat, der gleich ist wie ein Krümmungsradius der ventralen Fläche oder der dorsalen Fläche in der Draufsicht;
**dadurch gekennzeichnet, dass**
der Führungsschienenabschnitt derart ausgebildet ist, dass er in einen von dem Eingriffsabschnitt in der ventralen Fläche und dem Eingriffsabschnitt in der dorsalen Fläche eingepasst wird,
wobei einer von dem transversalen Querschnitt des Eingriffsabschnitts und dem transversalen Querschnitt des Führungsschienenabschnitts eine konkave Form hat, während der andere eine konvexe Form hat, die der konkaven Form entspricht, und die konkave Form einen Abschnitt mit einer maximalen Breite aufweist, welche größer ist als eine Öffnungsbreite der konkaven Form, die der konvexen Form gegenüberliegt.

2. System nach Anspruch 1, wobei der Führungsschienenabschnitt eine transversale Breite gleich der oder kleiner als die Höhe der ventralen Fläche und/oder der dorsalen Fläche des Zwischenkörperdistanzstücks hat.

3. System nach Anspruch 1 oder 2, **dadurch gekennzeichnet, dass** der transversale Querschnitt des Eingriffsabschnitts eine T-förmige Konkavität ist, und der transversale Querschnitt des Führungsschienenabschnitts eine T-förmige Konvexität ist.

4. System nach Anspruch 1 oder 2, **dadurch gekennzeichnet, dass** der transversale Querschnitt des Eingriffsabschnitts eine T-förmige Konvexität ist, und der transversale Querschnitt des Führungsschienenabschnitts eine T-förmige Konkavität ist.

5. Zwischenkörperdistanzstück nach einem der Ansprüche 1 bis 4, wobei der Eingriffsabschnitt einen Krümmungsradius in der Draufsicht hat, der gleich ist wie ein Krümmungsradius der ventralen Fläche oder der dorsalen Fläche in der Draufsicht.

6. Führungswerkzeug nach einem der Ansprüche 1 bis 4.

## Revendications

1. Système pour traiter une maladie rachidienne, ledit système comprenant :
un espaceur intervertébral qui est incurvé dans une direction horizontale et utilisé en étant inséré entre des corps vertébraux, l'espaceur intervertébral (10) incluant une paire de surfaces de contact destinées à être en contact avec chacun des corps vertébraux, et
incluant une surface ventrale qui est une surface latérale sur un côté ventral ayant une forme convexe en vue en plan et qui connecte ladite paire de surfaces de contact sur le côté ventral, et
une surface dorsale qui est une surface latérale sur un côté dorsal ayant une forme concave en vue en plan et qui connecte ladite paire de surfaces de contact sur le côté dorsal,
l'écarteur intervertébral incluant une partie d'engagement (20) qui est située le long de la direction dans laquelle l'écarteur intervertébral est incurvé, dans la surface ventrale et/ou la surface dorsale ;
et un outil de guidage (30) pour guider l'espaceur intervertébral vers une position prédéterminée entre les corps vertébraux,
l'outil de guidage incluant, sur un côté d'extrémité distale, une partie de rail de guidage (31),
ladite partie de rail de guidage ayant un rayon de courbure en vue en plan qui est le même qu'un rayon de courbure de la surface ventrale ou de la surface dorsale en vue en plan ;
**caractérisé en ce que**
la partie de rail de guidage est conçue de manière à être insérée dans l'une ou l'autre de la partie d'engagement dans la surface ventrale et de la partie d'engagement dans la surface dorsale,
dans lequel l'une ou l'autre de la section transversale de la partie d'engagement et de la partie transversale de la partie de rail de guidage présente une forme concave, tandis que l'autre présente une forme convexe qui correspond à la forme concave, et la forme concave inclut une partie ayant une largeur maximale qui est plus grande qu'une largeur d'ouverture de la forme concave qui est opposée à la forme convexe.

2. Système selon la revendication 1, dans lequel la partie de rail de guidage présente une largeur transversale inférieure ou égale à la hauteur de la surface ventrale et/ou la surface dorsale de l'écarteur intervertébral.

3. Système selon la revendication 1 ou 2, **caractérisé en ce que** la section transversale de la partie d'engagement est concave en T, et la section transversale de la partie de rail de guidage est convexe en T.

4. Système selon la revendication 1 ou 2, **caractérisé en ce que** la section transversale de la partie d'engagement est convexe en T, et la section transversale de la partie de rail de guidage est concave en T.

5. Écarteur intervertébral selon l'une quelconque des revendications 1 à 4, la partie d'engagement ayant un rayon de courbure en vue en plan qui est le même qu'un rayon de courbure de la surface ventrale ou de la surface dorsale en vue en plan.

6. Outil de guidage selon l'une quelconque des revendications 1 à 4.
